# EUROPÄISCHE PATENTSCHRIFT

(11) **EP 2 694 980 B1**
(45) Veröffentlichungstag und Bekanntmachung des Hinweises auf die Patenterteilung: **26.08.2015**
(21) Anmeldenummer: 12716301.2
(22) Anmeldetag: 04.04.2012
(51) Int. Cl.: G01N 33/82

(54) **BESTIMMUNG VON VITAMIN-D-METABOLITEN IN TROCKENBLUT**
ANALYSING VITAMIN D METABOLITES IN DRIED BLOOD
DÉTERMINATION DE MÉTABOLITES DE VITAMINE D DANS DU SANG SÉCHÉ

(30) Priorität: 04.04.2011 DE 102011001790
(43) Veröffentlichungstag der Anmeldung: 12.02.2014
(73) Patentinhaber: Immundiagnostik AG, 64625 Bensheim (DE)
(72) Erfinder: ARMBRUSTER, Franz Paul, 67240 Bobenheim-Roxheim (DE); GRÖN, Hans Jürgen, 64646 Heppenheim (DE); SCHUMANN, Claudia, 69469 Weinheim (DE)
(74) Vertreter: Benedum, Ulrich Max
(86) Internationale Anmeldenummer: PCT/EP2012/056204
(87) Internationale Veröffentlichungsnummer: WO 2012/136720

(56) Entgegenhaltungen:
- WO-A1-2008/092917
- WO-A2-2005/116081
- WO-A2-2006/107992
- US-A1- 2005 014 211

## Beschreibung

### GEBIET DER ERFINDUNG

Die Erfindung betrifft Verfahren zur Bestimmung von Vitamin-D-Metaboliten in Körperflüssigkeiten wie Blut Serum oder Milch.

### HINTERGRUND DER ERFINDUNG

Der Mensch kann Vitamin-D₃ (Cholecalciferol) mit Hilfe der UV-Strahlen im Sonnenlicht auf der Haut bilden. Vitamin-D₂ (Ergocalciferol) nimmt er über die Nahrung auf. Ergo- und Cholecalciferol unterscheiden sich in den Seitenketten und in ihrer biologischen Aktivität. Sie werden aber beide in der Zirkulation vom Vitamin-D-Bindungsprotein (VDBP) gebunden und in der Leber zu 25-Hydroxyvitamin-D (25(OH)D oder Calcidiol) metabolisiert (siehe Schmidt-Gayk H. et al (Hrsg.), Calcium regulation hormones, vitamin D metabolites and cyclic AMP, Springer Verlag, Heidelberg (1990), Seiten 24-47). 25(OH)D ist die Speicherform im Körper und im Blut der Vitamin-D-Metabolit mit der höchsten Konzentration. Bei Bedarf wird 25(OH)D in der Niere von der 25-Hydroxyvitamin-D-1α-hydroxylase zu 1α,25-Dihydroxyvitamin-D, dem D-Hormon oder Calcitriol hydroxyliert, welches die biologisch aktive Form ist. Die Aktivität der Vitamin-D-hydroxylase wird vielfältig reguliert, unter anderem durch das Parathormon und den Calcium-Spiegel im Blut. Das Calcitrol wird intrazellulär vom Vitamin-D-Rezeptor (VDR) gebunden und in den Zellkern transportiert. Dort assoziiert der Vitamin-D-Rezeptor-Komplex an die DNA, was zu Änderungen in der Proteinsynthese führt.

Die Bestimmung der Vitamin-D-Metaboliten in Blut oder Serum ist indiziert bei Verdacht auf Vitamin-D-Mangel, zum Beispiel bedingt durch zu geringe Synthese, reduzierter intestinaler Aufnahme und Maladsorption, Leberfunktionsstörungen, medikamentös erhöhtem Vitamin D-Stoffwechsel wegen der Einnahme von Antiepileptika, erhöhtem Vitamin D-Verlust aufgrund eines nephrotischen Syndroms oder einer allgemeinen Störung des Calcium-Phosphat-Gleichgewichts. In der sonnenlichtarmen Winterzeit leiden beispielsweise in Nordeuropa bis zu einem Drittel der älteren Normalbevölkerung an Vitamin-D-Mangel (Scharia et al., Osteoporose Int. 1998; 8(2):7-12). Ein leichter Mangelzustand von weniger als 15 Nanogramm Calcidiol pro Milliliter Serum (37,5 nMol/L 25-OH-Vit.D/L) verursacht wegen der verminderten Calciumaufnahme einen Parathormonanstieg und eine erhöhte Knochenresorption (Chapuy MC et al. J Clin Endocrinol Metab 1996; 81:1129-33). Vitamin-D-Mangel ist daher ein wichtiger Risikofaktor für die senile Osteoporose. Ein schwerer Mangel von weniger als 5 Nanogramm Calcidiol pro Milliliter Serum (12,5 nMol/L) bedingt bei Kindern Rachitis oder bei Erwachsenen Osteomalazie (Scharia et al. Exp Clin Endocrinol. Diabetes, 1996, 104:289-292). Ein Mangel Vitamin-D wird auch in Zusammenhang gebracht mit einem höheren Risiko für Brust-, Darm- und Prostatakrebs und verschiedenen Autoimmunerkrankungen wie juveniler Diabetes und Multipler Sklerose (Holick MF, Vitamin D deficiency. N Engl J Med (2007) 357:266-81). Eine Überdosierung von Vitamin-D verursacht das Hypercalcämie-Syndrom. Die Mindestkonzentation im Serum für Knochengesundheit liegt zwischen 20 und 32 ng 25(OH)D/mL (50-80 nmol/L) und eine Konzentration von mehr als 30 ng/mL beziehungsweise 75 nmol/L 25(OH)D ist anzustreben.

Die chemischen Verfahren zur Bestimmung der Vitamin-D-Metabolite sind aufwändig (siehe Tanner et al., J. Assoc. of Analyt. Chem. (1988) 17, 607-710; Haddad, J.G. et al., J Clin Endocrinol. Metab. (1971) 33, 992-995 and Eisman, J.A. et al., Anal. Biochem. (1977) 80, 298-305). US 5 981 779 (Holick et al), WO 89/01631 und EP 0 583 945 (DeLuca et al) lehren immunologische Bestimmungsverfahren. Vor der immunolo-gischen Bestimmung müssen die Proben sorgfältig aufbereitet werden, denn im Humanserum sind mehr als 85% der Vitamin-D-Metabolite an VDBP, Albumin und anderen Proteinen gebunden. WO 99/67211 (Armbruster et al) lehrt eine Ethanolfällung der Serumproteine und eine Analytik der Vitamin-D-Metabolite im ethanolischen Überstand. EP 0 753 743 (Hollis) empfiehlt eine Perjodatfällung der Serumproteine und eine Analytik im proteinfreien wässrigen Überstand. DE 10 144 905 C2 (Armbruster et al) lehrt eine Analytik direkt im Serum nach Freisetzung der Vitamin-D-Metabolite aus ihren Bindungen durch Zusatz von Verdrängungsmitteln wie Salizylsäure, Anilinosulfonsäure oder Warfarin. WO 2002/057797 (Quest Diagnostics Inc.) beansprucht den Zusatz von Salizylsäure, Cyclodextrin und einen pH-Wechsel für die Freisetzung der Vitamin-D-Metabolite. EP 2 126 586 B1 (Immundiagnostik AG) lehrt den Verdau der Serumproteine durch eine Serinprotease, gefolgt von einem Immunoassay an einem Protease-unempfindlichen monoklonalen Antikörper. Verschiedene Automatenverfahren unterziehen die Serumprobe einer Deproteinierung und Delipidierung mit Hilfe von Acetonitril. WO 2011/122948 (Future Diagnostics BV) offenbart die Freisetzung der Vitamin-D-Metabolite in der Serumprobe durch ein fluorhaltiges Tensid wie Perfluoroctansäure.

US 7,745,226 B2 (Clarke et al) lehrt eine Kombination von Flüssigkeitschromatographie und Massenspektroskopie für die Bestimmung der Vitamin-D-Metabolite im Serum. Die verschiedenen LC-MS, LC-SMS und LC-TMS werden nachstehend kurz als LC-MS-Verfahren bezeichnet (siehe Vogeser M, Liquid chromatography-tandem mass spectrometry · Application in the clinical laboratory, Clin Chem Lab Med (2003) 41:117-26; Vogeser M. Seger C. A decade of HPLC-MS/MS in the routine clinical laboratory - goals for further developments. Clin Biochem (2008) 41:649-62). Wenngleich die LC-MS-Analytik als der neue Standard in der Vitamin-D-Analytik gilt, so gibt es Kontroversen zur Zuverlässigkeit und zur Interpretation der Messwerte, da die Bezugswerte historisch durch Immunverfahren ermittelt wurden und alle Therapieempfehlungen auf ihnen beruhen. Die Immunverfahren geben in der Regel um circa 15 Prozent niedrigere Werte an als die LC-MS-Verfahren. Eine Umrechnung der Werte ist nicht möglich, zumal auch im Zeitraum 2007 bis 2008 viele der durch LC-MS im Serum bestimmten 25(OH)D-Spiegel sich später als zu hoch erwiesen (siehe Andrew Pollack in New York Times vom 7/8. Januar 2009). Untersuchungen mit dem automatisierten 25(OH)D-Assay von Roche zeigen, dass 25-Hydroxyvitamin-D₃ im Blut bei Raumtemperatur bis zu 3 Tagen stabil ist und im Serum sogar noch länger (Wielders et al, Clin Chem (2009) 55(8), 1584-5). Der 25(OH)D-Assay von Roche erfasst aber keine Medikation mit Vitamin-D₂ und die genannten Untersuchungen zur Temperaturstabilität von 25(OH)D₃ im Serum erfassen nicht das wirkliche präanalytische Geschehen in den Arztpraxen und Hospitälern. Auch der 25(OH)D-EIA von (Immunodiagnostic System Ltd) und dessen automatisierte Version (Siemens ADVIA Centaur Vitamin D total Assay) neigen zu systematischen Fehlern (siehe Cavalier et al, JBMR (2011) 26:434-436). Mit den derzeitigen LC-MS- und den Immunverfahren muss daher mit Fehlbestimmung gerechnet werden, die zu einer falschen Medikation führen, insbesondere wenn die Blut- uns Serumproben einer Hämolyse unterlagen. Der Stand der Technik repräsentiert daher ein Problem.

### ZUSAMMENFASSUNG DER ERFINDUNG

Das Problem wird durch das Verfahren nach Anspruch 1 gelöst. Bevorzugte Ausführungsformen des Verfahrens sind den abhängigen Ansprüchen zu entnehmen.

Die Erfindung betrifft die präanalytische Handhabung der Probe der Körperflüssigkeit (Blut, Serum, Plasma oder Milch) vor der Bestimmung der jeweiligen Vitamin-D-Metabolite und insbesondere die Bestimmung des Vitamin-D-Status (Total-Vitamin-D-Status), umfassend 25-Hydroxyvitamin-D₂, 25-Hydroxyvitamin-D₃, 1α,25-Di-hydroxyvitamin-D₂, 1α,25-Dihydroxy-Vitamin-D₃. Die Erfindung ist aber nicht auf diese speziellen Vitamin-D-Metabolite beschränkt.

Das Verfahren zur Bestimmung von Vitamin-D-Metaboliten, einzeln oder in Kombination, umfasst folgende präanalytische Schritte: (i) Bereitstellen eines festen Sorptionsmaterials, das eine vorgegebene Menge an Körperflüssigkeit, insbesondere Blut, physisch sorbieren kann, wobei das Sorptionsmaterial so gewählt ist, dass es von einer Behandlung mit protischen organischen Lösemitteln nicht angegriffen wird und keine analytisch störenden Substanzen freisetzt; (ii) Aufbringen einer vorgegebenen Menge Körperflüssigkeit, zum Beispiel Blut, auf das Sorptionsmaterial und Adsorption der Flüssigkeit auf dem Sorptionsmaterial; (iii) Lagern oder Transportieren des Sorptionsmaterials mit der sorbierten flüssigen Probe bis zur Bestimmung der Vitamin-D-Metabolite in geschütztem Zustand, wobei ein Schutz gegen Berührung, Feuchtigkeit und/oder Lichteinwirkung bereitgestellt wird. Ist die Erfindung am Beispiel von Vollblut, Serum, Plasma oder Kapillarblut beschrieben, so sind dies bevorzugte Beispiele, in denen eine Bestimmung von Vitamin-D-Metaboliten erfolgen kann. Wenngleich Serum oder Plasma per se keiner Hämolyse unterliegen, so können sie doch Spuren einer Hämolyse enthalten, insbesondere dann, wenn die Blutprobe bereits gealtert war oder falsch gehandhabt wurde oder einfach zu einer Hämolyse neigte.

Der Schutz gegen Berührung, Feuchtigkeit und Licht kann durch eine Hülle oder einen Umschlag oder eine Kassette für das Adsorptionsmaterial erfolgen. Bevorzugt ist das Sorptionsmaterial mit der Schutzvorrichtung lösbar verbunden und über einen Schlitz oder ein Loch für eine Kapillare oder eine Pipettenspitze zugänglich. Die Probe ist sorbiert auf einem Sorptionsmaterial über mindestens sieben Tage und länger bei Umgebungstemperatur für die Zwecke der quantitativen Bestimmung von Vitamin-D-Metaboliten stabil. Eine Hämolyse der Blutprobe auf dem Sorptionsmaterial stört die spätere Bestimmung der Vitamin-D-Metabolite in der Probe nicht. Anders Blutproben, die in einem Gefäß "gewöhnlich" hämolysiert sind. Wenn aus hämolysierten Blutproben Serum oder Plasma gewonnen und auf Vitamin-D-Metabolite untersucht wird, weichen die ermittelten Konzentrationen für die Vitamin-D-Metabolite signifikant ab. Offenbar können im flüssigen Zustand verschiedene bei der Hämolyse freigesetzte Stoffe sich mit den Vitamin-D-Metaboliten umsetzen. Es ist im Stand der Technik durchgängig beschrieben, dass bereits eine geringe Hämolyse stört. Es wurde zudem gefunden, dass präanalytisch die Vitamin-D-Metabolite, wenn in einem faserförmigen Sorptionsmaterial absorbiert, im Wesentlichen temperaturstabil sind. Offenbar kommen sie in diesem Zustand - sorbiert auf einem Adsorptionsmaterial - nicht mit katalytisch wirkenden Substanzen aus der Hämolyse zusammen und sie sind zudem durch das Material und die Hülle vor Licht geschützt. Auch können wässrige enzymatische Reaktionen nach Adsorption auf einem Sorptionsmaterial nicht erfolgen. Ohne durch eine Theorie gebunden sein zu wollen, wird angenommen, dass entweder die für eine Störung verantwortlichen Proteine und Enzyme dann auf dem Sorptionsmaterial auch partiell denaturiert vorliegen oder dass sorbiert auf der festen Oberfläche den Vitamin-D-Metaboliten einfach die Reaktionspartner für eine Umsetzung fehlen.

Die Präanalytik wird ergänzt durch folgende analytische Schritte: (i) Transferieren des festen Sorptionsmaterial mit der getrockneten Probe aus der Schutzvorrichtung in ein Gefäß; (ii) Zusetzen zur festen Phase einer Menge an wässrigem Lösepuffer, wobei der Lösepuffer einen pH zwischen 7,0 und 10,0 besitzt, ein oder mehrere Detergenzien und Tenside enthält und fakultativ 0,001 bis 100 mMol/L, bevorzugt 1 bis 20 mMol/L Cyanid-Ionen; (iii) Behandeln des festen Sorptionsmaterial mit der Probe mit dem wässrigen Lösepuffer, gegebenenfalls bei erhöhter Temperatur; und (iv) weiteres Zusetzen einer Menge einer organischen Elutionslösung, dass die flüssige Phase in dem Gefäß eine Permittivitätszahl zwischen 16 und 35 besitzt, bevorzugt zwischen 16 und 30, besonders bevorzugt von 22 bis 28, und Eluieren der Vitamin-D-Metabolite aus der festen Phase; (v) Abtrennen der festen Phase mit dem Adsorptionsmaterials und daran gebundenen und/oder präzipitieren Serum- und Zellproteinen aus der flüssigen Phase und (vi) Einsetzen eines Aliquots der flüssigen Phase mit den gelösten Vitamin-D-Metaboliten in die Analyse.

Solange die Probe der Körperflüssigkeit beziehungsweise die Blutprobe auf dem Sorptionsmaterial sorbiert ist, sind enzymatische und chemische Umsetzungen der Vitamin-D-Metabolite mangels Wasser oder Reaktionspartner oder beidem weitgehend unterbunden. Dies ändert sich mit Zusatz des wässrigen Lösepuffers, da dann eine flüssige Phase bereitsteht und eine eventuelle Hämolyse der Probe sich auswirken kann. Der Lösepuffer sollte deshalb Umsetzungen der Vitamin-D-Metabolite, mit anderen löslichen Bestandteilen des Blutes unterbinden beziehungsweise der Anlösevorgang kurz gehalten werden.

Der Lösepuffer kann Kaliumhexacyanoferrat(II) enthalten, eine Quelle für Cyanid-Ionen, PSMF, Detergentien und Tenside wie SDS, Triton X-100®; Perfluoralkylverbindungen, Perfluoralkansäure und übliche Mittel für eine Stabilisierung und zur Verhinderung von Mikrobenbefall.

Die wässrige Phase kann weiterhin Denaturierungsmittel und Verdrängungsmittel für das Lösen der Vitamin-D-Metabolite aus den Serumproteinen enthalten. Besonders bevorzugt sind Salizylat- und Salizylverbindungen, Warfarin, Sulfonate, Toluölsulfonate, Naphthalensulfonate, Anilinonaphthalensulfonate.

In einer Variante des Verfahrens kann die flüssige Phase für das Lösen der Vitamin-D-Metabolite eine Protease enthalten, welche die Proteine auf dem Sorptionsmaterial spaltet. Dies betrifft insbesondere den Verdau von Proteinen wie Albumin, Vitamin-D-Bindungsprotein (DBP) und Vitamin-D-Rezeptor (VDR), die bekanntlich Vitamin-D-Metabolite binden. Besonders bevorzugt ist.die Verwendung von Proteinase K. Proteinase K ist eine hochaktive Serinprotease aus Tritirachium album, das eine breite Spezifität für natürliche und denaturierte Proteine besitzt, und auch Proteine verdaut, wenn sie denaturiert auf einem hydrophoben Adsorptionsmaterial vorliegen. (Roelcke D & Uhlenbruck G, Z Med Mikrobiol Immunol. (1969) 155:156-170). Denaturierungsmittel wie Natriumdodecylsulphat (SDS) oder Harnstoff sowie erhöhte Temperaturen von 50 bis 60 Grad Celsius erhöhen seine Aktivität. Ein Konzentrationsbereich von 100 µg bis 2 mg/mL für die Behandlung des Adsorptionsmaterials ist empfohlen, eine Endkonzentration von 100 to 300 µg/mL im Lösepuffer bei 37 bis 50 Grad Celsius ist bevorzugt.

Der pH des Lösepuffers liegt bevorzugt zwischen 7.0 und 10.0, besonders bevorzugt zwischen 7.0 und 8.0. Ein bevorzugter Puffer zum Anlösen von Trockenblut auf dem Adsorptionsmaterial enthält 50 mMol/L NaH₂PO₄/Na₂HPO₄, pH 8.0, 1 % Gelatine, 10 mMol/L Cyanid-Ionen, 2 mMol/L EGTA, 1 mM β-Mercaptoethanol, 1 % SDS, 250 µg/mL Proteinase-K.

Die wässrige Phase kann wahlfrei bis zu 5 Gewichtsprozent, bevorzugt 1 bis 3 Gewichtsprozent natürliches und/oder chemisch modifiziertes Cyclodextrin enthalten, um störende Fettsäuren, Cholesterine und andere Lipide auszuschließen. Das Cyclodextrin kann chemisch modifiziert sein, beispielsweise Gruppen aufweisen wie Methyl, Ethyl, Propyl, Hydroxyethyl, 2-Hydroxypropyl, Glycosyl, Maltosyl, Carboxymethyl Natürliche Cyclodextrine und 2-Hydroxypropyl-β-cyclodextrin sind besonders bevorzugt. Diese Substanzen können auch im späteren lipophilen Elutionspuffer enthalten sein und in Schritten zugesetzt werden.

Insgesamt sind kurze Anlösezeiten der auf der festen Phase sorbierten Probe bevorzugt. Bevorzugt sind Anlösezeiten von 10 bis 300 Sekunden, bei einer Temperatur von Umgebungstemperatur bis 60°C, bevorzugt von Umgebungstemperatur bis 40°C.

Die protische organische Phase für das Eluieren der lipophilen Vitamin-D-Metabolite besitzt eine Permittivität ε kleiner 35, bevorzugt kleiner 30. Bevorzugt sind Gemische von Methanol und Propanol in einem Volumenverhältnis von 95 zu 5 bis 5 zu 95. Besonders bevorzugt ist ein Methanol-Isopropanol-Gemisch im Volumenverhältnis von 80 zu 20 bis 95 zu 5. Weitere geeignete protische Lösemittel für das Lösen der Vitamin-D-Metaboliten von dem Adsorptionsmaterial sind Propanol, Butanol, Isobutanol, Ethylenglycol, Acrylnitril, Acetonitril, Acrylester, Dimethylacetamid, Dimethylformamid, Dimethylsulfoxid, Beta-Ketoester, Methylethylketon, N-Methyl-2-pyrrolidon, N-Methylformamid, Isoamylalkohol, Nitrobenzol, Nitromethan, Acetoacetat, Perfluoralkansäuren, Perfluorsulfonsäuren, und deren Gemische, so dass die geeignete Permittivität über das Mischungsverhältnis erzielt wird.

Die feste Phase wird nach Elution der Vitamin-D-Metabolite abzentrifugiert und der Überstand mit den gelösten Vitamin-D-Metaboliten in die Analyse eingesetzt. Bei automatisierten Verfahren wird bevorzugt die festen Phase aus der Probe abfiltriert und das Filtrat mit den gelösten Vitamin-D-Metaboliten in die Analyse eingesetzt.

Die Bestimmung der Vitamin-D-Metabolite im Überstand oder Filtrat kann in einem kompetiven Bindungsassay erfolgen, bevorzugt in einem kompetitiven Bindungsassay auf der Basis von Antikörpern gegen den Vitamin-D-Analyt. Letzteres ist erforderlich, wenn beim Ablösen der Vitamin-D-Metabolite von der festen Phase Proteinase K eingesetzt wurde. Bevorzugte Verfahren des kompetiven Bindungsassays sind beschrieben in der WO 99/067211 der Anmelderin.

Der kompetitive Bindungsassay kann beispielsweise erfolgen in einem ELISA (enzyme-linked immuno-sorbens assay), RIA (radioimmunoassay), FIA (fluorescence immunoassay), LIA (luminescence immunoassay), oder ILMA (immuno luminometric assay). Der Analyt ist in der Regel 25-Hydroxyvitamin-D₂, 25-Hydroxyvitamin-D₃, 1α,25-Dihydroxyvitamin-D₂, 1α,25-Dihydrooy-Vitamin-D₃ (Calcitriol), sowie Vitamin-D₂ und D₃. Es können auch andere Vitamin-D-Metabolite bestimmt werden wie Vitamin-D₄ und D₅.

Alternativ kann die Bestimmung der Vitamin-D-Metabolite in der Analysenprobe durch Tandem-Massenspektroskopie erfolgen, wobei protonierte, hydratisierte und/oder dehydratisierte Ionen des Vitamin-D-Metaboliten in der Analysenprobe erzeugt werden und der Nachweis von Fragmenten davon für die Anwesenheit und die Menge der Vitamin-D-Metabolite in der Probe stehen. Die Probenvorbereitung für die Tandem-MS-Analytik kennt im Stand der Technik eine Elution von Trockenblutspots (Chase DH et al, Clin. Chem. (1993) 39:66-71). Hiermit ist aber nur eine qualitative Analyse von löslichen Aminosäuren möglich. Die quantiative Bestimmung von lipophilen Vitamin-D-Metaboliten aus Trockenblutspots war im Stand der Technik nicht bekannt.

Ein weiterer Aspekt der offenbarung betrifft einen Testkit für den präanalytischen Teil des Verfahrens zur quantitativen Bestimmung von Vitamin-D-Metaboliten in Blut, umfassend eine Kapillare für das Nehmen und Auftragen einer definierten Menge Blutprobe auf ein Adsorptionsmaterial; sowie eine Schutzvorrichtung wie einen Umschlag oder eine Kassette, enthaltend in geschütztem Zustand ein Sorptionsmaterial, das so saugfähig ist, dass die Menge an flüssiger Probe wirksam gebunden wird; das zudem bei der späteren Behandlung mit protischen organischen Lösungsmitteln nicht angegriffen wird und keine analytisch störenden Substanzen freisetzt; und das auf seiner Oberfläche Wechselwirkungen bereitstellt für eine Bindung von Proteinen auf dem Sorptionsmaterial; wobei das Sorptionsmaterial lösbar mit dem Umschlag oder der Kassette verbunden ist und mit der Kapillare benetzt werden kann. Dieser Testkit ist insbesondere gedacht für die Probennahme von Blut aus Fingerbeere, Ohr oder einer anderen üblichen Anstichstellen. Der Testkit erlaubt eine erleichterte Routinediagnostik, zum Beispiel für Heilpraktiker und kleine Arztpraxen, da die entnommenen Proben erleichtert transportiert werden können.

Das erfindungsgemäße analytische Verfahren bietet gegenüber früheren Verfahren den Messvorteil, dass störende Proteine nach Elution der Vitamin-D-Metaboliten - ohne Wechsel des Mediums - direkt durch Zentrifugation oder Filtration zusammen mit dem Sorptionsmaterial aus der Analysenprobe entfernt werden können. Weiterhin kann eine Bestimmung direkt aus Vollblut beziehungsweise Kapillarblut erfolgen. Eine Hämolyse auf dem Sorptionsmaterial stört die spätere Bestimmung nicht. Zudem besteht weniger Gefahr, dass im System Restmengen von Vitamin-D-bindenden Proteinen verbleiben, die bei einem Mediumwechsel renaturieren und die Bestimmung verfälschen. In den bisherigen Verfahren können nicht kontrollierbare Restmengen von Vitamin-D bindenden Proteinen an den Wänden der Probengefäße physisorbiert und vertragen werden, so dass sie bei einem Pufferwechsel die Vitamin-D-Metaboliten erneut binden und die Messergebnisse unvorhersehbar verfälschen. Ähnliches gilt auch für die hydrophoben Vitamin-D-Metabolite, die durch selektive Adsorption auf der Probengefäßwand aus der Probe verschwinden können. Auch unterscheiden sich die Gefäße für die Entnahme von Blutproben je nach Hersteller und können so Einfluss auf die Bestimmung nehmen. Dies ist für die praktische Reproduzierbarkeit von größter Bedeutung, was aber bei den herkömmlichen internen und externen Vergleichen zur Reproduzierbar regelmäßig nicht berücksichtigt wird. In diesen Vergleichsreihen werden regelmäßig gleiche Probengefäße oder zumindest Probengefäßes eines Herstellers verwendet. Man kann aber schwerlich Arztpraxen nur für die Bestimmung von Vitamin-D-Metaboliten auf das Probengefäß eines Herstellers festlegen. Erfindungsgemäß werden alle Zell- und Plasmaproteine präanalytisch auf ein und demselben getesteten Sorptionsmaterial gebunden. Das Problem der unkontrollierten Bindung auf den Wänden der unterschiedlichen Blutentnahmegefäße entfällt. Dadurch wird präanalytisch eine Fehlerquelle ausgeschaltet, die ansonsten bei allen anderen Verfahren weiterhin besteht. Daneben besteht der Vorteil einer sicheren Probenhandhabung, welche lange Transportwege toleriert.

Weitere Vorteile, Merkmale und Ausführungsformen der Erfindung sind der eingehenden Beschreibung der Erfindung, den Beispielen und den Abbildungen zu entnehmen.

### KURZE BESCHREIBUNG DER ABBILDUNGEN

Es zeigt:
- Fig.1A-D: Diagramme mit graphischen Darstellungen der Korrelation der gemessenen 25(OH)D-Konzentrationen in Serum und Vollblut nach unterschiedlich langer trockener Adsorption (1, 2, 3 und 7 Tagen) auf einem Sorptionsmaterial gemäß der Erfindung;
- Fig. 2: ein Diagramm mit einer graphische Darstellung der Korrelation der gemessenen 25(OH)D-Konzentrationen in Serum und Vollblut nach Hämolyse und ethanolischer Präzipitation der Serumproteine gemäß dem Stand der Technik;
- Fig.3A-C: Diagramme mit graphischen Darstellungen der Korrelation der gemessenen 25(OH)D-Konzentrationen in Kapillarblut (Blut aus der Fingerbeere), EDTA-Vollblut und Serum (venösem Blut aus der Armbeuge) nach unterschiedlich langer trockener Adsorption auf einem Sorptionsmaterial gemäß der Erfindung;
- Fig. 4: Diagramm mit einer graphischen Darstellung der Korrelation der gemessenen 25(OH)D-Konzentration in Kapillarblut (Blut aus der Fingerbeere) nach kurzzeitiger Adsorption auf einem Adsorptionsmaterial und zeitgleich parallel bestimmt in frisch gewonnenem Serum (venösem Blut aus der Armbeuge).

### EINGEHENDE BESCHREIBUNG DES ERFINDUNG

Zur quantitativen Bestimmung der Vitamin-D-Metabolite in einer Trockenblutproble gehört präanalytisch, dass die Menge an Blut für das Sorptionsmaterial genau abgemessen sein muss. Dies kann mit einer geeichten Kapillare erfolgen. Probenmenge und Menge des Sorptionsmaterials sind aufeinander abgestimmt. Eine geeignetes Verhältnis sind beispielsweise 50 bis 100 µl Blutprobe auf 200 bis 400 µl Sorptionsmaterial, wobei auch andere Verhältnissse möglich sind. Nach Auftragen der flüssigen Probe auf dem Sorptionsmaterial bei Umgebungstemperatur (0 bis 40 Grad Celsius) sind die Vitamin-D-Metabolite und Lichtschutz stabil. Hierzu befindet sich das Sorptionsmaterial bevorzugt in einer Schutzvorrichtung, in einer Kassette, einem Umschlag oder in einer sonstigen Hülle. Das Sorptionsmaterial ist bevorzugt durch eine kleine Öffnung oder einen Schlitz für eine Kapillare oder Pipette zugänglich und nach dem Auftragen der Probe wird die Blutflüssigkeit durch die Saugwirkung des Sorptionsmaterials in das Innere der Hülle gesogen. Sie ist dann für Versand, Transport und Lagerung vor Berührung, Feuchtigkeit, Schmutz und Licht geschützt.

Erfolgt die Bestimmung der Vitamin-D-Metabolite in Kapillarblut, wird zum Beispiel die Fingerbeere oder das Ohrläppchen angestochen, und nach Verwerfen des ersten Tropfens Gewebsflüssigkeit wird eine definierte Menge Blut (55% Plasma und ca 44% Erythrozyten Leukozyten, Thrombozyten) mit Hilfe einer Messkapillare aufgenommen und auf das saugfähige Sorptionsmaterial überführt. Dort verbleibt die Blutprobe bis zur Analyse. Das Sorptionsmaterial ist ein vliesartiges Membran- oder Filtermaterial, das Serumproteine (z.B. Albumin, Globuline) und Zellproteine durch hydrophobe Wechselwirkung an sich binden kann. Elektrolyte, niedrig-molekulare Stoffe wie Zucker, Lipide und Stoffwechselprodukte (Harnstoff, Harnsäure) fallen als Salze auf dem Material aus oder bleiben auf der Oberfläche adsorbiert. Entsprechendes gilt für die Blutfarbstoffe Hämoglobin (Hbll), Oxyhämoglobin (HbII-O₂), Hämiglobin (Hblll), Carboxyhämoglobin (HbII-CO). Das Sorptionsmaterial kann sein Cellulose, Nitrocellulose, Nylon, Mylar®, PVDF (Polyvinylidenfluorid) oder ein Material, wie es in der Mikrobiologie zum Westernblotting eingesetzt werden. Das Material sollte filter- oder filzartig sein und stark saugfähig sein. Das Adsorptionsmaterial ist bevorzugt flüssigkeitsundurchlässig.

Die Verwendung von Filtermaterial aus behandelter Cellulose oder Nitrocellulose ist bevorzugt, besonders bevorzugt ist ein Filtermaterial aus reiner Cellulose, Nitrocellulose, Nylon und/oder PVDF (Polyvinylidenfluorid), das resistent ist gegenüber üblichen organischen Lösemitteln. Prinzipiell geeignet sind Filtermaterialien geeignet, die in protischen organischen Lösemitteln nicht angegeriffen werden. Besonders bevorzugt sind Membranmaterialien, die Wechselwirkungen für die Bindung von Proteinen bereitstellen, zugleich aber auch lipophobe Eigenschaften besitzen. Nach dem Auftragen von Blut wird bei Vollblut in der Regel die Blutgerinnung bzw. die Hämolyse in Gang gesetzt. Auch Serum- und Zellproteine werden dann durch hydrophobe Wechselwirkungen auf dem Sorptionsmaterial gebunden.

Eine Hämolyse schadet nicht. Eventuell oxidiertes Hämiglobin (HbIII) wird von den Cyanid-Ionen im Anlösepuffer in das stabile HbIII-CN überführt. Bilirubin ist wasserunlöslich, und es wird im Blut als Bilirubin-Albumin-Komplex transportiert. Es verbleibt auf dem Sorptionsmaterial irreversibel. Restmengen könnten bei der Analytik im Immunoassay die Peroxidase-Farbbildung stören. Der Lösepuffer kann neben Detergenzien und der Cyanid-Quelle optional auch Kaliumhexacyanoferrat(III) enthalten für die Überführung von Hämoglobin(II) in Hämiglobin(III).

Für die Analytik müssen die Vitamin-D-Metabolite von der Membran abgelöst beziehungsweise desorbiert werden. Bei diesem Vorgang können die Proteine auf dem Sorptionsmaterial zum Teil renaturieren, ihre Sekundär- und Tertiärstruktur wieder einnehmen und die Vitamin-D-Metabolite an die Membran binden. Zudem werden die Vitamin-D-Metaboliten nicht nur vom Vitamin-D-Bindungsprotein (VDBP) gebunden, sondern auch von Serumproteinen wie Albumin, Fetoprotein, und so weiter, welche im Serum beziehungsweise auf dem Sorptionsmaterial abundant vorliegen. Durch das organische Elutionsmittel werden die Vitamin-D-Metabolite von der festen Phase desorbiert und in Lösung gebracht, während die Vitamin-D bindenden Proteine weitgehend auf der festen Phase oder gegebenenfalls auf Gefäßwände und anderen hydophoben Oberflächen übergehen. Diese könnten die Bestimmung verfälschen. Auch bei der LC-MS bestünde dieses Problem, denn in der Tandem-Massenspektroskopie kann nur bestimmt werden, was in der Probenlösung vorliegt. Im vorliegenden Fall ist es aber egal, ob die Vitamin-D bindenden Protein auf dem Sorptionsmaterial sind oder der Gefäßwand, denn die flüssig organische Phase mit den Vitamin-D-Metaboliten wird stets von der festen Phase getrennt beziehungsweise für die Bestimmung in ein anderes Gefäß überführt.

Besonders bevorzugt enthält der Lösepuffer Salizylat- und Salizylverbindungen oder Warfarin zum Verdrängen der Vitamin-D-Metabolite aus ihren Proteinbindungen. Der Einsatz von Verdrängungsmittel wie Salizyl und die Überführung der VitaminD-Metabolite von dem Adsorptionsmaterial in ein protisches, organisches Lösemittel mit einer Permittivität kleiner 30, bevorzugt kleiner 25 begegnet diesem Problem, da die störenden Proteine bereits durch Adsorption auf der Membran teilweise denaturiert sind, zum anderen durch das organische Elutionsmittel denaturiert werden und schließlich durch Zentrifugation oder Filtration aus der flüssigen Phase - ohne Änderung der Phase - zusammen mit dem Adsorptionsmaterial entfernt werden. Zudem sind Cholecalciferol und Ergocalciferol (Vitamin D₂ und D₃) und deren Derivate lipophile Verbindungen, die nur schwer von einem Sorptionsmaterial in eine wässrige Phase zu bringen sind. Das angegebene Elutionsmittel behebt dieses Problem.

In einer weiteren Ausführungsform umfasst das Verfahren zur Bestimmung von Vitamin-D-Metaboliten ein Zusetzen einer Serinprotease mit endo- und exoproteolytischer Aktivität in den Lösepuffer und den Verdau der Vitamin-D bindenden Proteine auf dem Adsorptionsmaterial und in der Lösung.

Ein Problem bei kolorimetischen Immunoassays ist deren Störanfälligkeit gegenüber Bilirubin, das konzentrationsabhängig in Gegenwart von Peroxidase H₂O₂ verbraucht und so verminderte Farbstoffausbeuten und falsche Messergebnis verursacht. Dieses Problem ist besonders bei der Bestimmung von niedrigen Analytkonzentrationen gegeben. Die Bilirubin-Interferenz kann reduziert werden durch Zusatz von Kaliumhexacyanoferrat(II) (K₄Fe(CN)₆]) zum Testreagenz, wobei Konzentrationen von ca. 5 bis maximal 50 µmol/L im Testansatz anzustreben sind; vgl. Nägele et al, Methods in Enzymatic Analysis (H.U. Bergmeyer, Hrsg.) 3. Ausgabe, Verlag Chemie, Weinheim 1985, Band VIII, Seiten 12-18). Der Lösepuffer für die Vitamin-D-Metabolite auf dem Adsorptionsmaterial (nicht zu verwechseln mit dem Testreagenz) kann neben einer Cyanid-Quelle vorteilhaft Kaliumhexacyanoferrat(III) (K₃Fe(CN)₆) enthalten. Das Kaliumhexacyanoferrat(III) wird bei der Oxidation von Hämoglobin zu Kaliumhexacyanoferrat(II) reduziert, das dann für eine Bilirubin-Entstörung sorgt.

Nach Abtrennen des Sorptionsmaterials und der Proteine kann eine Bestimmung der Vitamin-D-Metabolite erfolgen, wie zum Beispiel beschrieben in der WO 03/03391 der Anmelderin. In der Regel ist der Analyt ausgewählt aus 25-Hydroxyvitamin-D₂, 25-Hydroxyvitamin-D₃, 1α,25-Dihydroxyvitamin-D₂, 1α,25-Dihydroxy-Vitamin-D₃. Die Proteinbindungsanalyse und quantitative Bestimmung erfolgen bevorzugt in einem ELISA (Enzyme-linked immunosorbens assay), RIA (radioimmunoassay), FIA (fluorescence immunoassay), LIA (luminescence immunoassay), ILMA (immunolumino-metric assay) oder ECLA (electrochemical luminescence assay).

Die protische organische Phase mit den eluierten Vitamin-D-Metaboliten kann nach Zusatz von deuteriertem Vitamin-D-Metabolit als Standard auch durch Tandem-Massenspektroskopie analysiert werden. Hierbei geht der Analyse in der Regel eine zeit- und arbeitsintensive Flüssig-Flüssig-Extraktion oder eine manuelle Festphasenextraktion (SPE) zur Entfernung der Proteine voran. Diese Form der Probenvorbereitung kann entfallen. In der Regel wird eine einfache Trap-Säule vor der analytischen Säule genügen. Die Ionisierung der protonierten, hydratisierten und/oder dehydratisierten Precursor-Ionen kann durch APCI (atomosphereic pressure chemical ionization) oder ESI (electrospray ionization) erfolgen. Weitere denkbare Ionisationsverfahren umfassend Photoionisation, Elektronenionisation, FAB (fast atom bombardment), LSIMS (Liquid secondary ionization), MALDI (matrix assisted laser desorption ionization), Feldionisation und dergleichen. Die analytische Säule kann eine übliche HPLC (high performance liquid chromatography) sein. Die Bestimmung der Vitamin-D-Metabolite aus der gewonnenen Probe kann dann in vielfältiger Weise erfolgen, vergleiche Tsugawa et al, Determination of 25-Hydroxyvitamin D in Human Plasma Using HPLC-Tandem Mass Spectrometry, Analytical Chemistry (2005), 77:3001-3007; Watson D et al, Analysis of Vitamin-D and its Metabolites Using Thermospray Liquid Chromatography-Masspectrometry, Biomedical Chromatography (1991) 5:153-160; Kissmeyer A-M et al, Sensitive analysis of alpha,25-dihydroxyvitamin D3 in biological fluids by liquid chromatography-tandem mass spectrometry" J Chrom, (2001) 935:93-103; Maunsell et al. Routine isotope-dilution liquid chromatography-tandem, mass spectrometry assay for simultaneuos measurement of the 25-hydroxy metabolites of vitamins D2 and D3, Clin Chem (2005) 51:1683-1690; Yeung et al., Characterization of the metabolic pathway of 1,25-dihydroxy-16-ene vitamin D3 in rat kidney by on-line high performance liquid chromatography-electrospray tandem mass spectrometry, Biochem. Pharmac (1995). 49:1099-1110; Higashi et al. Simultaneuos determination of 25-hydroxyvitamin D2 and 25-hydroxyvitamin D3 in human plasma by liquid chromatography-tandem mass spectrometry employing derivatization with a Cookson-type reagent, Biol. Pharm. Bull. (2001) 24: 738-743; Odrozywolska et al. Convergent synthesis, chiral HPLC, and vitamin D receptor affinity of analogs of 1,25-dihydroxycholecalciferol, Chirality (1999) 11:249-255.

### BEISPIELE

### Beispiel 1 - Bestimmung des Vitamin-D-Status in Kapillarblut

### (i) Präanalytik

Es wurden geeichte Kapillarröhrchen und saugfähiges Adsorptionsmaterial (Chromatography-grade blotting filter paper mit PVDF-Membran von Millipore, Billerica, MA, US) bereitgestellt und darauf 50 µl Blutprobe aus Finger überführt. Der erste Tropfen Gewebsflüssigkeit nach dem Anstechen wurde verworfen. Als Vergleich diente der Vitamin-D-Status in der Zirkulation. 50 µl Kapillarblut wurde auf 200 µl hydrophobes Membranmaterial überführt und getrocknet. Das Membranmaterial wurde in einer Umhüllung eingeschlossen und vor Licht, Berührung und Feuchtigkeit geschützt. Die Blutprobe konnte so ohne Veränderung 7 und mehr Tage bei Umgebungstemperatur transportiert oder gelagert werden.

Das Membranmaterial mit dem Trockenblut-Spot wurde in ein 1,5 mL Eppendorf-Gefäß überführt. Es folgte (i) 10 Minuten Anlösen des Trockenbluts mit 100 µl Lösepuffer (50 mM Na/K-Phosphatpuffer, pH 8.0; 10 mM NaCl, 0,1% SDS, 100 mM Natriumsalizylat, 5 mM KCN) bei 37 Grad Celsius und (ii) Zusatz von 400 µl Methanol-Isopropanol-Gemisch (7:3) 30 Minuten Elution der lipidlöslichen Vitamin-D-Metaboliten bei Umgebungstemperatur unter Bewegung. Eine Hämolyse stört nicht. Bei stark lipämischen Proben wurde zur Erhöhung der Reproduzierbarkeit des Bindungsassays dem Elutionspuffer 2,5 Gewichtsprozent beta-Cyclodextrin zugesetzt. Das Membranmaterial wurde 10 Minuten bei 3000 G abzentrifugiert; das Pellet mit dem Membränmaterial und präzipitiertem denaturiertem Protein wurde verworfen. Der Überstand wurde im Weiteren auf 4 bis 8 Grad Celsius gekühlt und in die Analyse eingesetzt, oder bis zur Bestimmung bei -20°C eingefroren. Ein mehrfaches Einfrieren und Auftauen der Probe wurde vermieden.

### (ii) Analytik von 25-Hydoxyvitamin-D

Die quantitative 25(OH)D-Bestimmung erfolgte, sofern nicht anders angegeben, streng nach der Arbeitsanleitung des Herstellers (Immundiagnostik AG, Bensheim) für die quantitative Bestimmung von 25(OH)D in Humanserum.

Für die Herstellung der Streptavidin-beschichtenen Mikrotiterplatte wurde in die Vertiefungen einer Mikrotiterplatte zunächst jeweils 100 ng Streptavidin gegeben, gelöst in 200 µl 60 mMol Natriumhydrogencarbonat, pH 9,6 und die Platte über Nacht bei 4°C inkubiert. Die Streptavidin-Lösung wurde entfernt und die Vertiefungen fünfmal mit 200 µl Waschpuffer (50 mM/L Phosphatpuffer, pH 6,0 0,05% Tween-20) gewaschen. Dann wurde in eine jede Vertiefung 250 µl Blockpuffer (Phosphatpuffer pH 8,0 mit 0,5% Casein, 1% Gelatine, 1% Thimerosal) gegeben, eine Stunde bei Raumtemperatur inkubiert, der Blockpuffer entfernt und jede Vertiefung nochmals fünfmal mit je 200 µl Waschpuffer gewaschen. In die Vertiefungen wurde dann jeweils 10 ng Biotin-25(OH)D-Tracer (25-OH-Vit.-D₃-3ß-3'[6-N-(biotinyl)hexamido]amidopropylether; WO 99/067211 A1) in 200 µl Waschpuffer gegeben, eine Stunde bei Raumtemperatur im Dunkeln und unter Rütteln inkubiert, die 25(OH)D-Tracer-Lösung aus den Vertiefungen entfernt und jede Vertiefung 5 mal mit je 200 µl Waschpuffer gewaschen. Es folgte in flüssiger Phase die Bindung von einem weitgehend Proteinase-K resistenten monoklonalen Maus-Antikörper gegen 25(OH)D₂/D₃ in Gegenwart von 25(OH)D aus Standard oder Analysenprobe (in Elutionspuffer).

Im Einzelnen wurde 40 µl Probe (Analysenprobe oder Standard) mit 360 µl Assaypuffer (50 mM K/Na-Phosphatpuffer, pH 8.0, 2 mM KCl, 10 mM NaCl, 2 Gew.% PEG-5000, 1% Gelatine) verdünnt und jeweils 200 µl für die kompetitive Bindungsanalyse in die Vertiefungen einer Streptavidin-beschichteten Mikrotiterplatte gegeben. Das Gemisch wurde dann 3 Stunden bei 4°C im Dunkeln unter Bewegung in der Vertiefung der Mikrotiterplatte inkubiert. Das in der Probe vorhandene 25(OH)D₂/D₃ konkurrierte mit dem Biotin-25(OH)D₃-Tracer um die Bindungsstelle am Antikörper, wobei Tracer-Antikörper-Komplex von der festen Phase in die Lösung abgeht. Danach wurden die Assaylösung aus den Vertiefungen entfernt und die Vertiefung fünfmal mit 200 µl Waschpuffer (50 mM K/Na-Phosphatpuffer, pH 8.0, 2 mM KCl, 10 mM NaCl, 1% Triton X-100®) gewaschen.

Für die Bestimmung der kompetitiven Bindung wurde 200 µl Konjugat-AK (Peroxidase-Kaninchen-Anti-Maus-mAB-Konjugat) in die Vertiefung gegeben und eine halbe Stunde bei Raumtemperatur im Dunkeln und unter Bewegung inkubiert. Die Lösung wurde entfernt und die Vertiefung fünfmal mit je 200 µl Waschpuffer gewaschen. Für die Farbreaktion wurden 100 µl Tetramethylbenzidin(TMB)-Substratlösung (NOVUM Diagnostika GmbH, Dietzenbach, DE) in die Vertiefungen gegeben. Nach 30 Minuten wurde die Farbentwicklung gestoppt durch Zugabe von 50 µL 2 M H₂SO₄ pro Vertiefung.

Als Standard wurden Lösungen von 25(OH)D₃-Assaypuffer mit folgenden Konzentrationen eingesetzt, 0, 6.4, 16, 40, 100 und 250 nMol/L (siehe Eichkurve). Als Kontrollen (Ka = 29,0 nMol 25(OH)D/L, Kb = 75,0 nMol 25(OH)D/L)) wurden Serum- und Blutproben aus einem Normalkollektiv eingesetzt, deren jeweilige Konzentration an 25(OH)D aus anderweitigen Bestimmungen bekannt war. In den Diagrammen mit den Eichkurven ist auf der Ordinate jeweils die optische Dichte als Mittelwert von zwei Messungen bei 450 nm aufgetragen; die Abszisse gibt die bestimmte Konzentration an 25(OH)D nMol/L an; siehe

### Beispiel 2 - Reproduzierbarkeit und Stabilität der Trockenblut-Probe

Werden Trockenblutproben mit der Post oder per Kurier an das Labor geschickt, dann müssen die Proben auch nach unterschiedlich langer Zeit die gleichen Werte für die Vitamin D-Metabolite in dem jeweiligen Testsystem ergeben. Der Versand von Laborproben per Post oder Kurier dauert in der Regel nicht länger als sieben Tage, da auch spätestens der Arzt oder Patient ein zeitiges Ergebnis erwartet.

Es wurden 50µl-Trockenblutproben auf jeweils 200µl Membranmaterial gemäß Beispiel 1 erstellt und für unterschiedliche lange Zeiten (1 bis 7 Tage) bei Umgebungstemperatur aufbewahrt. Danach wurden die getrockneten Vollblutproben auf dem Membranmaterial gemäß Beispiel 1 mit jeweils 100 µl Lösepuffer angelöst und 30 Minuten bei 37°C mit einem Methanol-Isopropanol-Gemisch einer Permittivität 25 behandelt. Dabei erfolgt die vollständige Ablösung der Vitamin-D-Metabolite von der Membran und eine Denaturierung der Plasma- beziehungsweise der Serumproteine, insbesondere des Vitamin-D-Bindungsproteins. Das Membranmaterial und denaturierte Plasma- und Serumproteine wurden durch Zentrifugation (3000 G / 10 Min) abgetrennt und 50 µl Überstand als Analysenprobe eingesetzt. Die Standardreihe (Serum auf Membran) wurden gleichbehandelt.

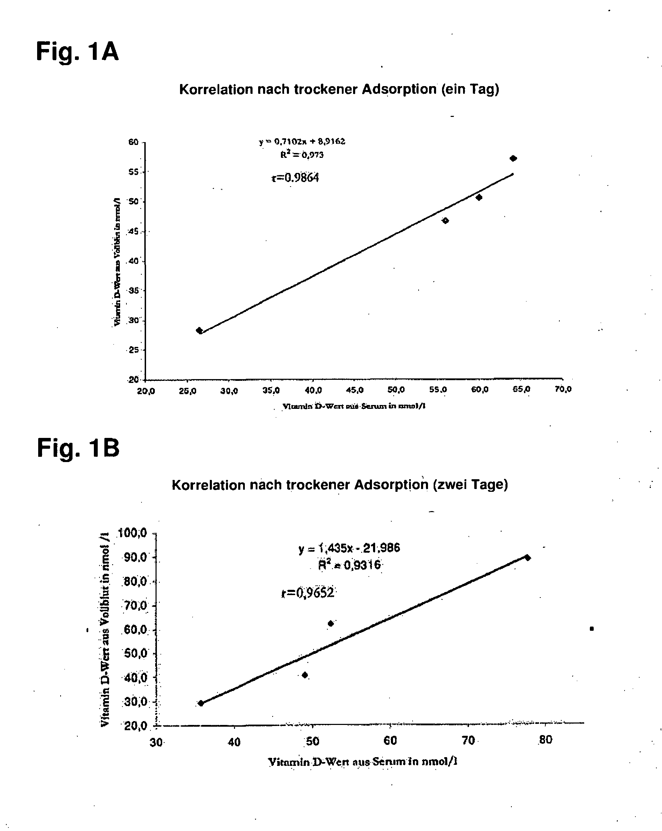

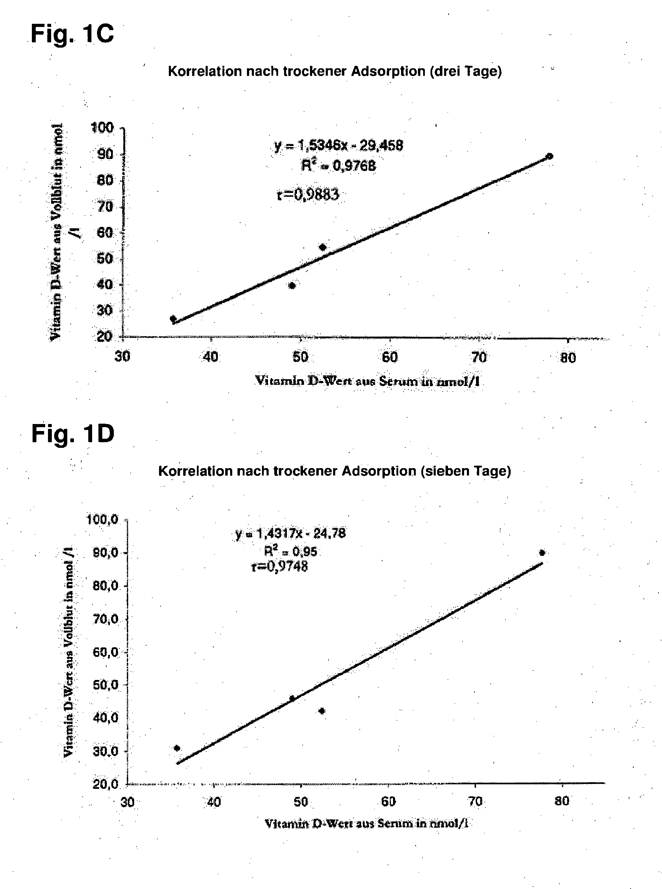

Die Analytik der Proben auf 25(OH)D erfolgte mit dem in Beispiel 1 angegebenem Testsystem. Der Korrelationswert r zwischen den Messwerten für 25(OH)D in Serum oder Vollblut betrug nach einem Tag Lagerung der Probe auf dem Membranmaterial 0.9864 (1,0 steht für vollständige Übereinstimmung), nach zwei Tagen r = 0,9652, nach drei Tagen r = 0,9883 und nach sieben Tagen r = 0,9748; siehe Figuren 1A-D. Die Länge der Lagerung der Probe auf dem Membranmaterial hatte somit keinen Einfluss auf die Korrelation der Messwerte; das Bestimmtheitsmaß R² entspricht der üblichen Pipettiervarianz einer manuellen Analytik.

### Beispiel 3 (Vergleichsbeispiel) - Bestimmung von 25(OH)D in Vollblut.

50 µl Standard (Standardreihe) beziehungsweise Serum und Vollblut des gleichen Patienten wurden in 1,5 ml Reaktionsgefäße pipettiert. Auf jeweils eine Zugabe von 450 µl Fällungsreagenz gemäß WO 99/067211, Mischen und 30 Minuten Inkubation bei -20°C folgte ein Abtrennen des Präzipitats durch Zentrifugation bei 3000 G / 4°C /10 Min. Es wurde jeweils 50 µl Überstand (Analysenprobe) mit 100 µl Assaypuffer in die Vertiefungen einer vorbereiteten Streptavidin-beschichteten 96er-Multititerplatte mit Biotin-25(OH)D-Tracer und Bindeprotein (Anti-25(OH)D-Ak) gemäß Beispiel 1 übertragen und 3 Stunden bei 8°C inkubiert. Die Vertiefungen der Platte wurden fünfmal mit 200 µl Waschpuffer gewaschen, 200 µl Peroxidase-Anti-Maus-IgG-Antikörper-Konjugat zugesetzt, nach einer Stunde erneut fünfmal gewaschen und dann der Test entwickelt. Die nachstehenden Tabelle I stellt die erhaltene Messwerte von acht Serum- und Vollblutproben gegebenüber.

**Tabelle I**

| Ergebnisse für den Vergleich Serum mit Vollblut in nmol/L | | |
|---|---|---|
| | Serum | Vollblut |
| Probe 1 | 82,7 | 67,9 |
| Probe 2 | 51,2 | 48,7 |
| Probe 3 | 78,8 | 74,8 |
| Probe 4 | 35,4 | 52 |
| Probe 5 | 89,6 | 70,8 |
| Probe 6 | 57,6 | 46,9 |
| Probe 7 | 51,0 | 45,0 |
| Probe 8 | 115,7 | 70,8 |

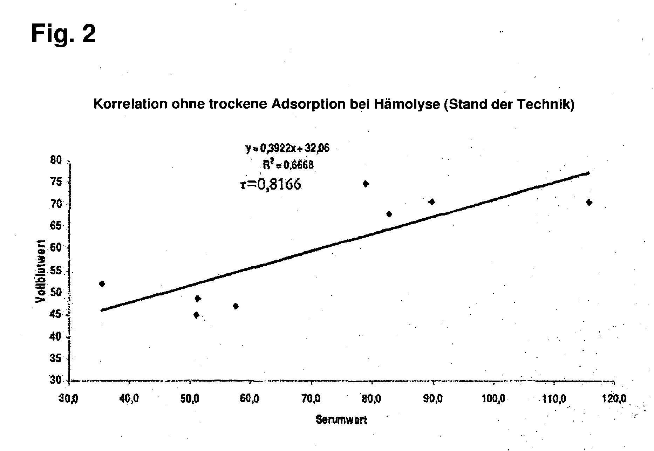

Die Tabelle zeigt, dass die 25(OH)D-Bestimmungen nur oft korrelieren, dass aber für einige Proben die aus Vollblut ermittelten Werte diagnostisch erheblich von den aus Serum ermittelten Werten abweichen. Mal sind die in Vollblut ermittelten Konzentration um die Hälfte zu hoch, mal um die Hälfte zu niedrig. Zwar beträgt der Korrelationswert r zwischen den Messwerten in Serum und Vollblut ca. 0,8 (siehe Figur 2), die Messungen erlauben aber keine therapeutischen Maßnahmen.

Eine Bestimmung von Vitamin-D-Metaboliten aus Vollblut ist für die Diagnostik mit den etablierten Verfahren nicht möglich. Es ist im Vollblut etwas vorhanden, das bei einer Hämolyse der Probe in nicht vorhersehbarer Weise die Konzentrationsbestimmung der Vitamin-D-Metaboliten in der Probe stört.

### Beispiel 4 - Vergleich der Bestimmungen von 25(OH)D in Kapillarblut gegenüber EDTA-Vollblut und Serum sowie zwischen Vollblut und Serum

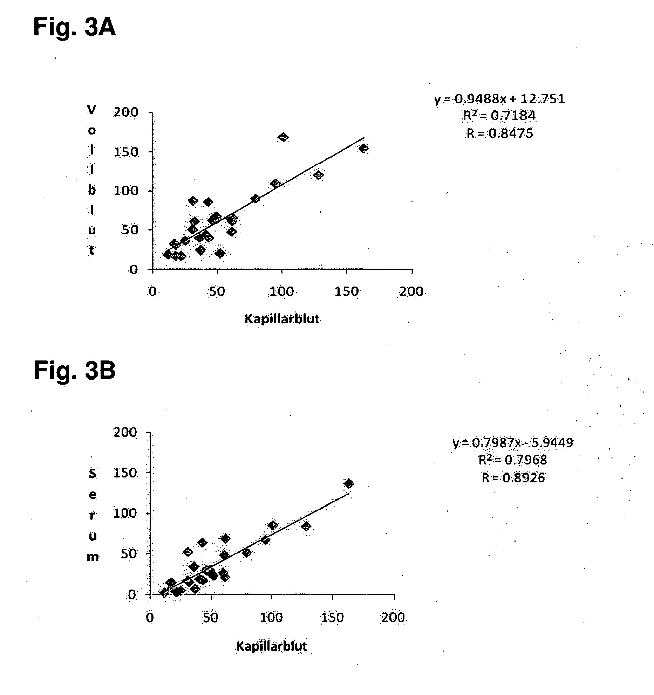

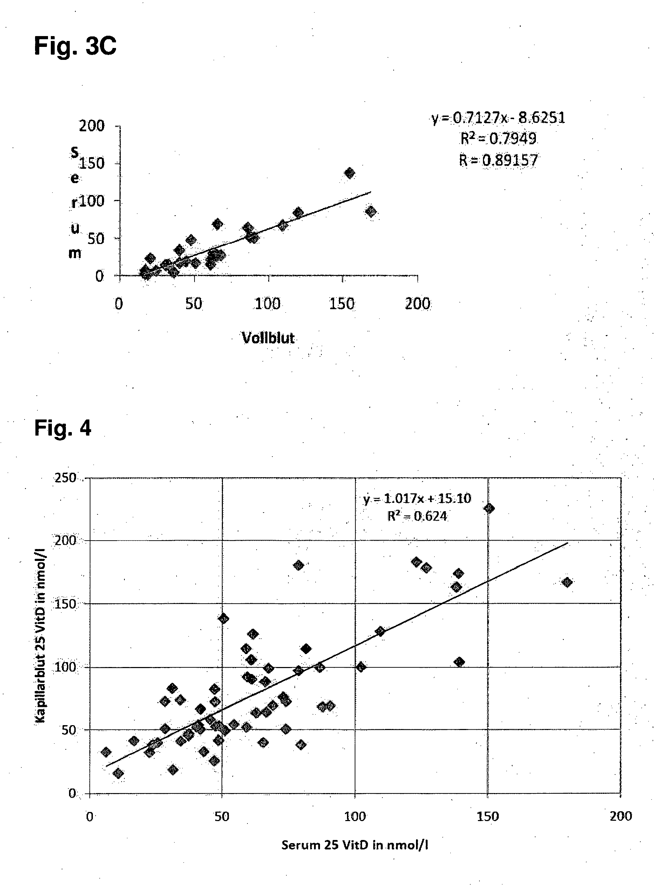

Von 25 Probanden wurde jeweils 50 µl Kapillarblut aus Fingerbeere sowie EDTA-Vollblut und Serum aus der Armbeuge auf 200 µl Sorptionsmaterial mit einer Kapillare übertragen und nach Trocknen auf dem Sorptionsmaterial am Folgetag wie in Beispiel 1, Absatz (ii) bestimmt. Die Resultate sind in Figuren 3A-C zusammengefasst. Figur 1A bestätigt, dass die Konzentrationen von 25(OH)D in den peripheren Blutgefäßen der Fingerkuppe nicht abweicht zur Konzentration im EDTA-Vollblut aus der Armbeuge. Kapillarblut lieferte in Bezug auf 25(OH)D jedenfalls keine andere Werte als EDTA-Vollblut. Gleiches gilt im Wesentlichen für die 25(OH)D-Bestimmung aus Serum, wobei hier es die durch die Gewinnung des Serums veränderte Volumenmenge zu berücksichtigen gilt. Durch die Gewinnung von Serum tritt ein "Verdünnungseffekt" auf, der sich auf die 25(OH)D-Konzentration unmittelbar auswirkt. Das Bestimmtheitsmaß R² zeigt, dass es bei der Bestimmung aus Trockenblut entscheidend auf die sorbierte Flüssigkeitsmenge ankommt. Variationen in der mit der Kapillare übertragenen Menge an Kapillarblut auf das Sorptionsmaterial beträchtigen die Bestimmtheit der Messung.

### Beispiel 5 - Vergleichsreihe der Bestimmungen von 25(OH)D in frischem Kapillarblut gegenüber frischem Serum aus der Armbeuge

Von 60 Probanden wurde jeweils Kapillarblut aus der Fingerbeere sowie Serum aus der Armbeuge gewonnen. 50 µl Kapillarblut wurde auf ein saugfähiges lipophobes Cellulose-Material aufgetragen. Zeitgleich wurde von Blut aus der Armbeuge Serum gewonnen und 50 µl Serum mit 950 µl Fällungsreagenz (siehe WO 99/67211) versetzt und die Serumproteine gefällt.

Es wurde 50 µl ethanolischer Überstand aus der Ethanolfällung der Serumprotein in die immunologische Bestimmung eingesetzt..

Für die Bestimmung aus Kapillarblut wurde das Sorptionsmaterial in 1,5 ml Reaktionsgefäße gegeben, 5 Minuten mit jeweils 100 µl Lösepuffer angelöst und 10 Minuten bei 37°C mit 900 µl Methanol-Isopropanol-Gemisch einer Permittivität 25 behandelt. Das Membranmaterial mit dem anhaftenden Plasma- und Serumprotein wurde durch Zentrifugation abgetrennt und 50 µl Überstand in die immunologische Bestimmung eingesetzt.

Die Bestimmung auf 25(OH)D erfolgte in beiden Fällen mit dem in Beispiel 1 angegebenem Testsystem. Die Ergebnisse sind Figur 4 zu entnehmen. Die Korrelation zwischen den Messreihen für 25(OH)D in Kapillarblut und Serum war mit 1,017 nahezu perfekt. Die vergleichsweise geringe Bestimmtheit (R²) von 0,624 beruhte auf der Überführung varianter Mengen Kapillarblut auf das Sorptionsmaterial, wie Vergleiche mit den Mengen an Hämoglobin in der Probe zeigten. Es empfiehlt sich daher bei Trockenblutbestimmungen zeitgleich die Menge an Hämoglobin in der Probe mit zu bestimmten, um so die Menge besser normieren zu können. Die Hämolyse der Kapillarprobe auf dem Sorptionsmaterial störte in keinem Fall die Bestimmung.

## Patentansprüche

1. Verfahren zur Bestimmung von Vitamin-D-Metaboliten in einer Probe einer Körperflüssigkeit, insbesondere in Blut oder einer Blutfraktion, deren Gehalt an Vitamin-D-Metaboliten zu bestimmen ist, umfassend die präanalytischen Schritte:
(i) Bereitstellen einer vorgegebenen Menge eines saugfähigen festen Sorptionsmaterials, das eine Probe der Körperflüssigkeit nach Auftrag adsorbieren kann; das so gewählt ist, dass es von der Behandlung mit protischen organischen Lösungsmitteln mit einer Permittivität kleiner 35 nicht angegriffen wird und keine analytisch störenden Substanzen freisetzt; und das auf seiner Oberfläche hydrophobe Wechselwirkungen bereitstellt für eine Bindung von Proteinen;
(ii) Aufbringen einer vorgegebenen Menge an Körperflüssigkeit auf das Sorptionsmaterial und Adsorbieren und Trocknen der flüssigen Probe auf dem Sorptionsmaterial, so dass die in der Probenflüssigkeit enthaltenen Proteine auf dem Sorptionsmaterial festgehalten sind;
(iii) Lagern oder Transportieren des Sorptionsmaterials mit der sorbierten flüssigen Probe bis zur quantitativen Bestimmung der Vitamin-D-Metabolite, in geschütztem Zustand, wobei ein Schutz gegen Berührung, Feuchtigkeit und/oder Lichteinwirkung bereitgestellt wird; und
(iv) Eluieren der Vitamin-D-Metabolite aus dem Sorptionsmaterial und Einsetzen in die Analyse.

2. Verfahren zur Bestimmung von Vitamin-D-Metaboliten gemäß Anspruch 1, zudem **gekennzeichnet durch** folgende analytische Schritte:
(i) Transferieren des festen Sorptionsmaterials mit der sorbierten Körperflüssigkeit aus dem Schutz in ein Gefäß;
(ii) Zusetzen zur festen Phase einer Menge an wässrigem Lösepuffer, wobei der wässrige Lösepuffer einen pH zwischen 7,0 und 10,0 besitzt und 0,01 bis 10 Gew.% Detergens und/oder Tensid enthält;
(iii) Behandeln der festen Phase mit der Probe mit dem wässrigen Lösepuffer bei einer Temperatur von Umgebungstemperatur bis 60°C für mindestens 10 Sekunden; und
(iv) Zusetzen einer Menge einer protischen organischen Elutionslösung, so dass die flüssige Phase in dem Gefäß eine Permittivität kleiner 35 besitzt und Eluieren der Vitamin-D-Metabolite in die flüssige Phase;
(v) Abtrennen der festen Phase mit gebundenen und/oder präzipitieren Serum- und Zellproteinen von der flüssigen Phase; und
(vi) Einsetzen eines Aliquots der flüssigen Phase mit den gelösten Vitämin-D-Metaboliten in die Analyse.

3. Verfahren nach Anspruch 2, wobei die flüssige organische Phase für das Eluieren der Vitamin-D-Metabolite eine Permittivitätszahl ε zwischen 16 und 30 besitzt.

4. Verfahren nach Anspruch 2 oder 3, wobei die flüssige Phase für das Überführen der Vitamin-D-Metabolite in die flüssige Phase Denaturierungsmittel und Verdrängungsmittel enthält, ausgewählt aus Salizylat- und Salizylverbindungen, Warfarin, Sulfonaten, Toluolsulfonaten, Naphthalensulfonaten, Anilinonaphthalensulfonaten.

5. Verfahren nach einem der Ansprüche 2 bis 4, wobei die flüssige Phase eine Serinprotease enthält.

6. Verfahren nach einem der Ansprüche 1 bis 5, wobei die Bestimmung, der Vitamin-D-Metabolite in der Analysenprobe in einem kompetitiven Bindungsassay erfolgt, bevorzugt in einem kompetitiven Bindungsassay auf Basis von Antikörpern gegen den Vitamin-D-Analyten.

7. Verfahren nach einem der Ansprüche 1 bis 5, wobei die Bestimmung der Vitamin-D-Metabolite in der Analysenprobe durch Tandem-Massenspektroskopie erfolgt, wobei protonierte, hydratisierte und/oder dehydratisierte Ionen des Vitamin-D-Metaboliten erzeugt werden und der Nachweis von Fragmenten davon für die Anwesenheit und die Menge von Vitamin-D-Metaboliten in der Probe stehen.

8. Verfahren nach einem der Ansprüche 1 bis 7, wobei der zu bestimmende Vitamin-D-Metabolit ausgewählt ist aus 25-Hydroxyvitamin-D₂; 25-Hydroxyvitamin-D₃, 1α,25-Dihydroxyvitamin-D₂, 1α,25-Dihydroxy-Vitamin-D₃.

9. Verfahren nach einem der vorstehenden Ansprüche 1 bis 6 oder 8, wobei die Proteinbindungsanalyse erfolgt in einem ELISA (enzyme-linked immunosorbens assay), RIA (radioimmunoassay). FIA (fluorescence immunoassay), LIA (luminescence immunoassay), oder ILMA.

10. Verfahren nach einem der vorstehenden Ansprüche 1 bis 9, wobei die Menge der sorbierten Probe der Körperflüssigkeit über einen einfach zu bestimmenden Referenzstoff und/oder über die Menge an sorbierten Hämoglobin mitbestimmt wird.

11. Verfahren zur quantitativen Bestimmung von Vitamin-D-Metaboliten in Blut gemäß Anspruch 1, zudem umfassend die präanalytischen Schritte:
(i) Bereitstellen eines Umschlages, einer Kassette oder einer Hülle;
(ii) Bereitstellen einer vorgegebenen Menge eines festen Sorptionsmaterials, das eine Blutprobe nach Auftrag adsorbieren kann; und
(iii) Bereitstellen einer Kapillare für das direkte verlustfreie Nehmen und Auftragen einer definierten Menge Blut auf das Sorptionsmaterial;
wobei das Sorptionsmaterial so saugfähig ist,
dass die Menge an aufgebrachter flüssiger Probe auf der festen Phase sorbiert wird,
dass bei der späteren Behandlung von protischen und organischen Lösungsmitteln mit einer Permittivität kleiner 35 nicht angegriffen oder angelöst wird, so dass keine störenden Substanzen freigesetzt werden, und
dass auf seiner Oberfläche Wechselwirkungen bereitstellt für eine Bindung von Proteinen auf dem Sorptionsmaterial;
und das Sorptionsmaterial lösbar so mit der Hülle, der Kassette oder dem Umschlag verbunden ist und mit der Kapillare benetzt werden kann, dass die Blutprobe während Lagerung und Transport vor Licht, Schmutz und Berührung geschützt ist.

12. Verfahren zur Bestimmung von Vitamin-D-Metaboliten in Blut gemäß Anspruch 1 für die Probennahme von Kapillarblut aus Finger oder Ohr.

## Claims

1. Method for determining vitamin D-metabolites in a sample of a body fluid, in particular in blood or a blood fraction, of which the content in vitamin D-metabolites should be determined, comprising the pre-analytical steps of:
(i) providing a predetermined amount of a bibulous solid sorption material capable of adsorbing a sample of the body fluid after application; the sorption material having been selected for not becoming attacked by a treatment with protic organic solvents having a permittivity lower than 35, and for not releasing any analytically interfering substances; and which on its surface provides hydrophobic interactions for a binding of proteins;
(ii) applying a predetermined amount of body fluid onto the sorption material and adsorbing and drying of the liquid sample onto the sorption material so that the proteins contained in the sample liquid are bound to the sorption material;
(iii) storing or transporting the sorption material with the sorbed liquid sample until the quantitative determination of vitamin D metabolites under protective conditions, wherein a protection against contact, moisture and/or light exposure is provided; and
(iv) eluting the vitamin D-metabolites from the sorption material and introducing into the analysis.

2. Method for determining vitamin D-metabolites according to claim 1, further **characterised by** the following analytical steps:
(i) transferring the solid sorption material with the sorbed body fluid from the protection into a vessel;
(ii) adding to the solid phase an amount of aqueous solvent buffer, wherein the aqueous solvent buffer has a pH between 7.0 and 10.0 and contains 0.01 to 10 wt.-% of a detergent and/or surfactant;
(iii) treating the solid phase with the sample with the aqueous solvent buffer at a temperature from ambient temperature to 60°C for at least 10 seconds; and
(iv) adding an amount of a protic organic elution solution so that the liquid phase in the vessel has a permittivity below 35, and eluting of the vitamin D-metabolites into the liquid phase;
(v) separating the solid phase with bound and/or precipitated serum and cell proteins from the liquid phase; and
(vi) introducing an aliquot of the liquid phase with the dissolved vitamin D-metabolites into the analysis.

3. Method according to claim 2, wherein the liquid organic phase for eluting of the vitamin D-metabolites has a permittivity number between 16 and 30.

4. Method according to claim 2 or 3, wherein the liquid phase for transferring the vitamin D-metabolites into the liquid phase contains denaturing and displacing agents selected from salicylate and salicylic compounds, warfarin, sulfonates, toluene sulfonates, naphthalene sulfonates, aniline naphthalene sulfonates.

5. Method according to one of claims 2 to 4, wherein the liquid phase contains a serine protease.

6. Method according to one of claims 1 to 5, wherein the determination of vitamin D-metabolites in the analytical sample is carried out in a competitive binding assay, preferably in a competitive binding assay based on antibodies against the vitamin D-analyte.

7. Method according to one of claims 1 to 5, wherein the determination of vitamin D-metabolites in the analytical sample is carried out using tandem mass spectroscopy, wherein protonated, hydrated and/or dehydrated ions of the vitamin D-metabolite are generated and the detection of fragments thereof represents the presence and amount of the vitamin D-metabolites in the sample.

8. Method according to one of claims 1 to 7 wherein the vitamin D-metabolite to be determined is selected from 25-hydroxyvitamin D₂, 25-hydroxyvitamin D₃, 1α,25-dihydroxyvitamin D₂, 1α,25-dihydroxyvitamin D₃.

9. Method according to one of the preceding claims 1 to 6 or 8, wherein the protein binding assay is carried out using an ELISA (enzyme-linked immunosorbens assay), RIA (radioimmunoassay), FIA (fluorescence immunoassay), LIA (luminescence immunoassay) or ILMA.

10. Method according to one of the preceding claims 1 to 9 wherein the amount of the sorbed body fluid sample is co-determined via an easy to determine reference material and/or the amount of sorbed haemoglobin.

11. Method for the quantitative determination of vitamin D-metabolites in blood according to claim 1, further comprising the pre-analytical steps of:
(i) providing an envelope, a cassette or a sleeve;
(ii) providing a predetermined amount of a solid sorption material, which may adsorb a blood sample after application; and
(iii) providing a capillary for the direct taking and applying of a determined amount of blood onto the sorption material, without loss;
wherein the sorption material is so bibulous,
that the amount of applied liquid sample is sorbed onto the solid phase; that with a later treatment of protic and organic solvents with a permittivity below 35 it is not attacked or solved, such that no interfering substances are released, and
that on its surface interactions are provided for a binding of proteins onto the sorption material;
and the sorption material is connected releasably with the sleeve, the cassette or the envelope and may be wetted with the capillary, in such a way that the blood sample is protected during storage and transport from light, dirt and contact.

12. Method for determining vitamin D-metabolites in blood according to claim 1 for the sample taking of capillary blood from the finger or the ear.

## Revendications

1. Méthode pour la détermination des métabolites de la vitamine D dans un échantillon d'un fluide biologique, en particulier dans le sang ou une fraction sanguine, dont il s'agit de déterminer la teneur en métabolites de vitamine D, comprenant les étapes pré-analytiques suivantes :
(i) préparation d'une quantité prédéterminée d'une matière de sorption solide absorbante qui peut adsorber un échantillon du fluide biologique après l'application ; qui est choisie de telle sorte qu'elle ne soit pas attaquée par le traitement avec des solvants organiques protiques d'une permittivité inférieure à 35 et ne libère pas de substances perturbant l'analyse ; et qui offre à sa surface des interactions hydrophobes pour la liaison aux protéines ;
(ii) application d'une quantité prédéterminée de fluide biologique sur la matière de sorption et adsorption et séchage de l'échantillon liquide sur la matière de sorption, de sorte que les protéines contenues dans le fluide échantillon soient retenues dans la matière de sorption ;
(iii) stockage ou transport de ma matière de sorption avec l'échantillon li-quide sorbé jusqu'à la détermination quantitative des métabolites de vitamine D sous protection avec mise à disposition d'une protection contre les contacts, contre l'humidité et / ou contre l'exposition à la lumière ; et
(iv) élution les métabolites de vitamine D de la matière de sorption et les introduire dans l'analyse.

2. Méthode pour la détermination des métabolites de vitamine D selon la revendication 1, par ailleurs **caractérisée par** les étapes analytiques suivantes :
(i) transfert de la matière de sorption solide avec le fluide biologique sorbé protégé dans un récipient ;
(ii) ajout à la phase solide d'une quantité de tampon en solution aqueuse, où le tampon en solution aqueuse présente un pH compris entre 7,0 et 10,0 et contient 0,01 à 10 % en poids de détergent et / ou de tensioactif ;
(iii) traitement de la phase solide avec l'échantillon au moyen du tampon en solution aqueuse à une température ambiante de 60°C au maximum pendant au moins 10 secondes ; et
(iv) addition d'une quantité d'une solution d'élution organique pro-tique, de sorte que la phase liquide dans le récipient atteigne une permittivité inférieure à 35 et élution des métabolites de vitamine D dans la phase liquide ;
(v) séparation de la phase solide avec la phase liquide à l'aide de pro-téines sériques et cellulaires liées et / ou précipitées ; et
(vi) utilisation d'un aliquote de la phase liquide avec les métabolites de vitamine D dissous dans l'analyse.

3. Méthode selon la revendication 2, où la phase organique liquide pour l'élution des métabolites de vitamine D présentant un indice de permittivité compris entre 16 et 30.

4. Méthode selon l'une quelconque des revendications 2 ou 3, où la phase liquide pour le transfert des métabolites de vitamine D dans la phase liquide contient des agents de dénaturation et de déplacement, sélectionnés dans des composés de salicylate et de salicyle, de warfarine (coumaphène), de sulfonates, de toluène sulfonates, de naphtalène sulfonates, d'anilinonaphthalène sulfonates.

5. Méthode selon l'une quelconque des revendications 2 à 4, où la phase liquide contient une protéase à sérine.

6. Méthode selon l'une quelconque des revendications 1 à 5, où la détermination des métabolites de vitamine D dans l'échantillon d'analyse est effectuée dans un dosage par liaison compétitive, de préférence dans un dosage par liaison compétitive sur la base d'anticorps contre les analytes de vitamine D.

7. Méthode selon l'une quelconque des revendications 1 à 5, où la détermination des métabolites de vitamine D dans l'échantillon d'analyse est effectuée par spectrométrie de masse en tandem, où se trouve une génération d'ions proto-nés, hydratés et / ou déshydratés des métabolites de vitamine D et où la preuve de fragments sont testés pour la présence et la quantité de métabolites de vitamine D.

8. Méthode selon l'une quelconque des Revendications 1 à 7, où un métabolite de vitamine D à déterminer est sélectionné parmi la 25-hydroxyvitamine-D₂, la 25-hydroxyvitamine-D₃, la 1α,25-dihydroxyvitamine-D₂, la 1α,25-dihydroxyvitamine-D₃.

9. Méthode selon l'une quelconque des Revendications 1 à 6 ou 8, où l'analyse de liaison des protéines s'effectue dans ELISA (Méthode immuno-enzymatique ELISA), RIA (radioimmunoessai), FIA (immunoessai de fluorescence), LIA (immunoessai de luminescence) ou ILMA.

10. Méthode selon l'une quelconque des revendications 1 à 9, où la quantité de l'échantillon sorbé du fluide biologique est déterminée en même temps par une matière de référence à déterminer et / ou par la quantité d'hémoglobine sorbée.

11. Méthode pour la détermination quantitative des métabolites de vitamine D dans le sang selon la revendication 1, comprenant en outre les étapes pré-analytiques :
(i) Préparation d'une enveloppe, d'une cassette ou d'une enveloppe ;
(ii) Préparation d'une quantité prédéterminée d'une matière de sorption solide qui peut adsorber un échantillon de sang ; et
(iii) Préparation d'une capillaire pour le prélèvement et l'application sans pertes d'une quantité définie de sang sur la matière de sorption ;
où la matière de sorption est suffisamment absorbante
pour absorber la quantité d'échantillon liquide appliquée sur la phase so-lide,
qu'il n'y ait pas d'attaque ou de corrosion lors du traitement ultérieur de solvants protiques et organiques avec une permittivité inférieure à 35, pour qu'aucune substance interférente ne soit dégagée et
qu'il n'y ait pas d'interactions sur la surface pour la liaison de protéines sur la matière de sorption ;
et que la matière de sorption soit détachable de l'enveloppe, de la cassette ou de l'enveloppe et puisse être aspergée avec la capillaire, que l'échantillon de sang soit protégé contre la lumière, les salissures et le contact pendant le stockage et le transport.

12. Méthode pour la détermination des métabolites de vitamine D dans le sang selon la revendication 1 pour la prise d'échantillons de sang capillaire sur le doigt ou sur l'oreille.
